Europäisches Patentamt

European Patent Office

Office européen des brevets

(19)

(11) Publication number : **0 483 014 A1**

(12) # EUROPEAN PATENT APPLICATION

(21) Application number : **91402859.2**

(22) Date of filing : **25.10.91**

(51) Int. Cl.⁵ : **G01N 33/50, G01N 33/573, G01N 33/94**

(30) Priority : **26.10.90 IT 4172090**

(43) Date of publication of application :
**29.04.92 Bulletin 92/18**

(84) Designated Contracting States :
**AT BE CH DE DK ES FR GB GR IT LI LU NL SE**

(71) Applicant : **FIDIA S.p.A.**
**Via Ponte della Fabbrica 3-A**
**I-35031 Abano Terme (Padova) (IT)**

(72) Inventor : **Della Valle, Francesco**
**Via Cerato 14**
**I-35100 Padova (IT)**
Inventor : **Dal Toso, Roberto**
**Viale dei Ciliegi 42**
**I-36040 Sovizzo, Vicenza (IT)**
Inventor : **Leon, Alberta**
**Piazza Napoli 19**
**Padova (IT)**

(74) Representative : **Hirsch, Marc-Roger et al**
**Cabinet Hirsch 34 rue de Bassano**
**F-75008 Paris (FR)**

(54) **A diagnostic method for idiopathic Parkinson's disease.**

(57)    The present invention is directed to an in vitro method for diagnosing Parkinson's disease in a human patient, which comprises the steps of :
   (a) culturing cells in the presence of dopamine and serum of a patient to be tested for Parkinson's disease ; and
   (b) measuring high-affinity dopamine uptake by said cells, and kits containing the same.

FIGURE 1

EP 0 483 014 A1

EP 0 483 014 A1

The present invention is directed to a method for the qualitative and quantitative determination of the presence of a complement-dependent humoral immune response in the sera of patients with idiopathic Parkinson's disease, by an in vitro biological test which measures high affinity dopamine uptake, in cultures of rat mesencephalic dopaminergic neurons.

This method can be used for diagnostic purposes in neurodegenerative pathological disorders such as idiopathic Parkinson's disease (Parkinson's disease, PK) and for the identification of new pharmacologically active molecules. The innovative quality of the method makes early diagnosis of idiopathic Parkinson's disease possible in sera from patients in whom Parkinson's disease has not yet induced any neurological alterations which can be detected by diagnostic methods currently described in the literature.

DESCRIPTION OF RELATED ART

Parkinson's disease (PK) is a neurodegenerative disorder involving a loss of dopaminergic neurons of the substantia nigra (SN). This loss is considered to be the fundamental lesion characterizing PK, even though little is known about its causes and relative mechanisms. Moreover, evidence from various sources (Calne D. B.: Is "Parkinson's disease" one disease? J. Neurol. Neurosurg. Psychiat. special suppl. 18-21, 1989; Sage J. I. et al.: Clinically atypical expression of pathologically typical Lewy-body parkinsonism. Clinical Neuropharm. 13: 36-47, 1990; Jellinger K.: Pathology of parkinsonism. In: Recent development in Parkinson's disease. Raven Press, 33-66, 1986) points to the fact that there are various mechanisms which cause a parkinsonism syndrome, so it has been proposed that the term "Parkinson's disease" should be substituted with "Idiopathic parkinsonism".

Recently, cells presenting antigens and T lymphocytes have been observed in the substantia nigra of PK patients, (McGeer P. L. et al.: Reactive microglia are positive for HLA-DR in the substantia nigra of Parkinson's and Alzheimer's disease brains. Neurology, 38: 1285-1291, 1988; McGeer P. L. et al.: Comparison of neuronal loss in Parkinson's disease and aging. In: Parkinsonism and aging. Raven Press, 25-34, 1989); on the basis of these findings, it has been postulated that the autoimmune mechanisms may play a role in the events leading to progressive dopaminergic neuron loss. Concordant with these results, it has been reported that the sera (Pouplard A. et al.: Parkinsonism and autoimmunity: antibody against human sympathetic ganglion cells in Parkinson's disease. Adv. Neurol. 24: 321-326, 1979) and cerebrospinal fluid (CSF) (McRae Degueurce A. et al.: An antibody in the CSF of Parkinson's disease patients disappears following adrenal medulla transplantation. Neurosci. Lett. 94: 192-197, 1988) of PK patients produce evident immunocytochemical staining in the sympathetic ganglia and in the mesencephalic region, respectively of the rat. In particular, earlier papers on antineuronal antibodies in the blood or CSF of PK patients do not report any specificity or direct effects of said antibodies on discrete neuronal populations; indeed, Pouplard et al. (Pouplard A. et al.: Parkinsonism and autoimmunity: antibody against human sympathetic ganglion cells in Parkinson's disease. Adv. Neurol. 24: 321-326, 1979) observed only the presence of circulating antibodies specific to a cytoplasmic component of the sympathetic ganglion neurons in sera from 62.7% of PK patients. It has also been observed that (McRae Degueurce A. et al.: An antibody in the CSF of Parkinson's disease patients disappears following adrenal medulla transplantation. Neurosci. Lett. 94: 192-197, 1988; Dahlstrom A. et al. Investigations on autoantibodies in Alzheimer's and Parkinson's diseases, using defined neuronal cultures. J. Neurol. Transm. 29: 195-206, 1990) samples of CSF from PK patients who have undergone an adrenal medulla transplant produce immunocytochemical staining in both the compact and reticular regions of rat substantia nigra, and they exercise general toxicity on mesencephalic cultures from 3-week-old rats (where most cells are glial, i.e. positive to GFAP).

However, despite these findings, the ability of antineuronal autoantibodies present in the blood or CSF to interact with neuronal surface antigens and produce complement-dependent lysis of intact dopaminergic neurons has never been demonstrated. Clearly, the role of humoral immune factors in the pathophysiology of parkinsonism is still open to speculation and must be clearly defined. It is well known that antibody-mediated cell damage can result from various mechanisms (Leibowitz S. et al.: Hypersensitivity in the nervous system. Immunol. Nerv. System. 1: 41-71, 1983), including cell-or complement-dependent cytotoxicity, or interaction with transmitter receptors.

An earlier study, carried out in Fidia Laboratories (Leon A. et al. Sera from parkinsonian patients contain inhibitory activity for development of fetal mesencephalic dopaminergic neurons in culture. J. Neurochem. 44:S 137 A, 1985), led to the suggestion that in PK patients a humoral response capable of damaging the dopaminergic neurons may be involved. It was observed that sera from PK patients contained a heat-sensitive agent which inhibited the development and survival of embryonic dopaminergic neurons in dissociated cocultures of striatal-mesencephalic neurons in the absence of serum. Sera from PK patients however had no effect on the behaviour of GABAergic cells, and similarly control sera from healthy volunteers was totally ineffective in inhibi-

2

ting DAergic and GABAergic cell survival.

Nowhere therefore, in the scientific information available to date, has a valid method ever been described for the early diagnosis of idiopathic Parkinson's disease, at a stage when the disorder cannot yet be recognized by its typical neurological symptoms.

## SUMMARY OF THE INVENTION

The present invention is directed to a method for qualitatively and quantitatively determining the presence of a complement-dependent humoral response in the sera of patients suffering from idiopathic Parkinson's disease, by an in vitro biological test which measures the specific dopamine uptakp on mesencephalic dopaminergic neuronal cultures of rat or mouse.

A further aspect of the invention is directed to a test kit comprising a first receptacle containing labeled dopamine and a second receptacle containing complement.

By the present invention it is demonstrated that serum from idiopathic Parkinson's disease patients presents complement-dependent inhibition of dopamine uptake (test specific for survival/viability of DAergic neurons), contrary to that of symptomatic parkinsonian patients and other neurological or normal control patients.

The possibility of specifically identifying at a very early stage a marker representative of a pathological situation in the CNS, provides a valuable diagnostic tool for the early identification of a neurodegenerative disease such as idiopathic Parkinson's disease, characterized by severe serous-complement-dependent cytotoxicity in DAergic neuronal cultures, and it opens up new preventive measures and consequent therapies for these pathologys.

## BRIEF DESCRIPTION OF THE DRAWINGS

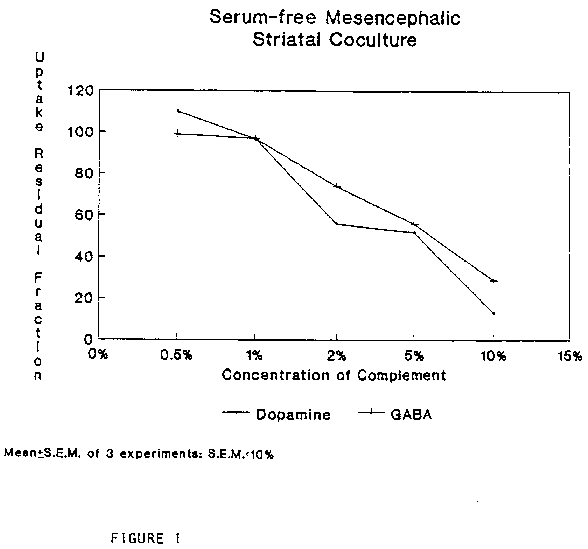

Figure 1 shows the effect of the concentration on high-affinity uptake of $^3$H dopamine and $^{14}$C GABA in mesencephalic-striatal cocultures. The mesencephalic and striatal cells were seeded at a density of $0.5 \times 10^6$ cells/well (24 mm) in 0.6 ml of serum-free culture medium. The complement and the inactivated complement were added "in vitro" on the 5th day 60 min before assessment of the uptake parameter. Values represent the mean ± S.E.M. of the residue fractions (RF) relative to 2 different experiments, each performed in triple determination. The standard error mean (S.E.M.) was less than 10% in all cases.

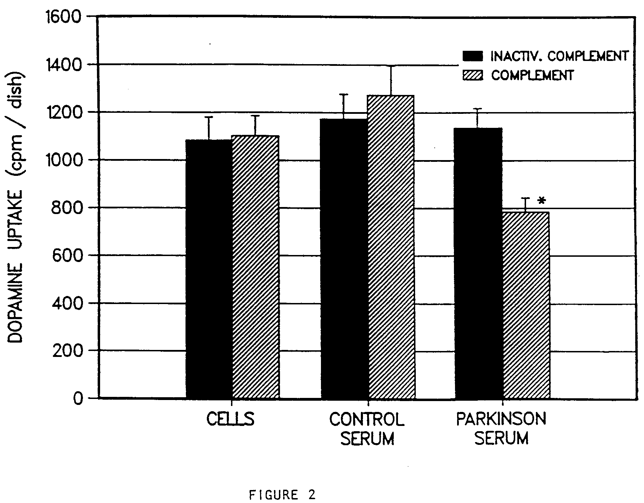

Figure 2 shows the effect of the complement (concentration 1% v/v) on high-affinity $^3$H dopamine uptake in mesencephalic striatal cocultures containing inactivated serum samples (concentration 5%) from one patient with Parkinson's disease and one neurologic control. Values represent the mean cpm ± S.E.M. of experiments in triplicate, each performed with triple determination. S.E.M. was less than 10% in all cases. Student's paired t-test: * p < 0.01 versus inactivated complement.

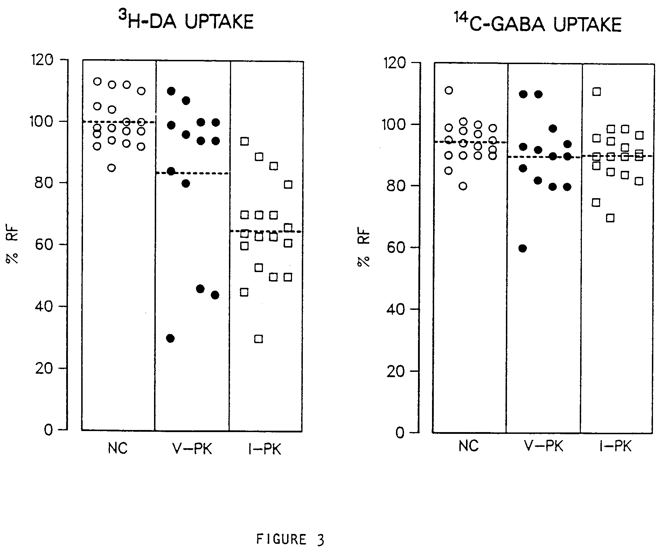

Figures 3A and 3B show the effect of complement (1% concentration on specific high-affinity $^3$H-dopamine and $^{14}$C-GABA uptakes in mesencephalic-striatal cocultures containing serum samples (5% concentration) from patients with idiopathic (I-PK; 18 patients) and vascular (V-PK, 13 patients) parkinsonism or other neurological diseases (NC: 18 patients). Values represent the mean residual fraction of each tested serum obtained from 3 different experiments, each analyzed in triplicate. The 100% value is the average of DA-RF and GABA-RF obtained in cultures added with serum and rabbit complement, both heat-inactivated.

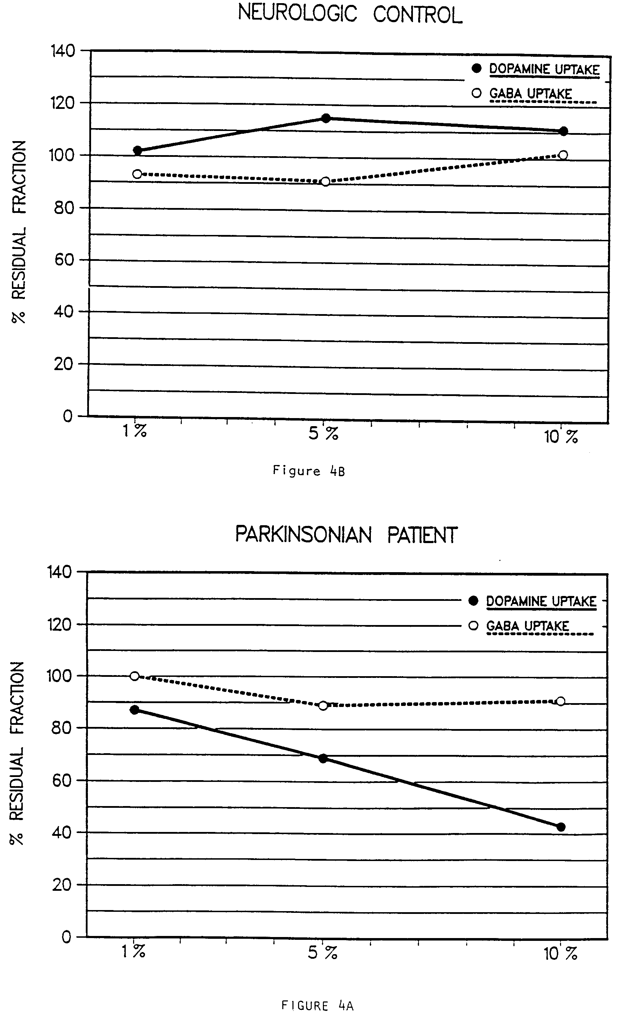

Figures 4A and 4B show the effect of concentration of heat-inactivated serum from one seropositive parkinsonian patient (A) and one neurologic control (B), on specific $^3$H-dopamine and $^{14}$C-GABA uptakes in mesencephalic-striatal cocultures. One percent reconstituted rabbit complement was present in all dishes. Values represent the mean residual fraction of three experiments, each performed in triplicate. S.E.M. was at all times less than < 10%.

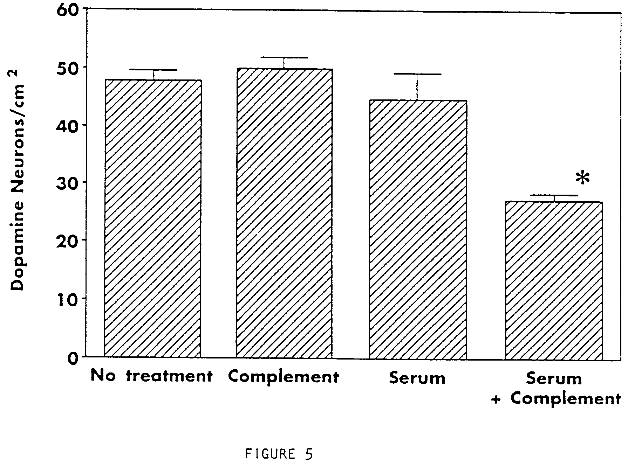

Figure 5 shows the effect of complement (1% concentration) and heat-inactivated serum (5% concentration) from a seropositive parkinsonian patient on TH immunoreactive cells in mesencephalic-striatal coculture. Values represent the mean ± S.E.M. number of TH containing cells per cm$^2$ from three coverslips. Students's paired t-test: * p < 0.01 vs. all other groups.

## DETAILED DESCRIPTION OF THE PREFERRED EMBODIMENTS

What follows is a detailed description of how the object of the present invention is accomplished. This description is for illustrative purposes only, and is not to be considered to limit the invention in any way, and any variation which would be evident to an expert in the art is to be considered as coming within the scope of the invention.

The following example refers to experiments conducted on sera obtained from parkinsonian patients (both idiopathic and symptomatic) and from neurological controls, tested after being inactivated (in a dilution range of 1 to 10%) on rat or mouse mesencephalic-striatal cocultures (on the 4th day) with subsequent exposure (24 hr later for 60 min) to + inactivated rabbit complement (56°C for 30 min). Specific uptake parameters were assessed (DA and GABA uptake, as specific, functional markers of dopaminergic and GABAergic neuron viability) and immunocytochemical parameters (TH immunofluorescence, as a specific marker for cells containing tyrosine hydroxylase, a key enzyme in dopamine synthesis).

Complement useful in carrying out the method of the present invention include complement obtained from rabbit or guinea pig.

The mesencephalic neuron cell cultures useful in carrying out the method of the present invention can be from any origin, for example, from louse, rat, guinea pig, bovine or human. Preferred mesencephalic neuron cell cultures can be obtained from the mouse or rat since they are easier and more economical to obtain.

In particular, hereinfater are described the materials and methods used to carry out the present example:

Patients

Samples of serum from 31 parkinsonian patients (both idiopathic and symptomatic) and 18 neurological controls were used.

Idiopathic parkinsonism was diagnosed in 18 patients, while the remaining 13 patients showed evidence of cerebrovascular disease (multi-infarct encephalopathy of the cerebral hemispheres and/or of the diencephalic medulla oblongata region) on analysis by computerized axial tomography (CAT) or nuclear magnetic resonance (NMR). Serum was taken from patients affected by neurological diseases other than PK to serve as controls (7 Alzheimer, 5 cerebrovascular disease, 2 blepharospasm, 2 hemifacial spasm, 1 Huntington's chorea, 1 amyotrophic lateral sclerosis). Demographic and clinical data of the two PK patient groups are reported in Table 1.

Blood samples were left to coagulate at room temperature for 60 min, separated by centrifugation and left at 70°C for no longer than 3 months. They were examined blind for cytotoxicity after being inactivated at a temperature of 56°C for 30 min.

## Table 1:

### Clinical data of the study groups

| | Parkinsonian Patients | |
|---|---|---|
| | Idiopathic | Vascular |
| Sample Size | 18 | 13 |
| Males/Females | 12/6 | 7/6 |
| Mean Age (yrs) $\pm$ SEM | 61 $\pm$ 2.5 | 65.4 $\pm$ 3.8 |
| Mean Age at onset (yrs) $\pm$ SEM | 53.7 $\pm$ 2.7 | 57.6 $\pm$ 3.1 |
| Mean Duration of illness (yrs) $\pm$ SEM | 7.4 $\pm$ 1.2 | 7.6 $\pm$ 1.5 |
| Clinical Features | | |
| Tremor | 4 | 2 |
| Rigidity | 4 | 3 |
| Complete Form | 11 | 8 |
| Hoehn Yahr Staging | | |
| I - II | 2 | 3 |
| III | 8 | 6 |
| IV - V | 8 | 4 |

Chi square test (2 x 2) with Yates correction: N.S. Mann Whitney non-parametric test: N.S.

Mesencephalic-striatal Cocultures

Rat striatal mesencephalic cocultures, according to Prochiantz et al. (Prochiantz A. et al.: In vitro maturation of mesencephalic dopaminergic neurons from mouse embryos is enhanced in presence of their striatal target cells. Proc. Natl. Acad. Sci. USA 76: 5387-5391, 1979) and described in detail by Dal Toso et al. (Dal Toso R. et al.: Development and survival of neurons in dissociated fetal mesencephalic serum-free cell cultures: effect of cell density and of an adult mammalian striatal-derived neuronotrophic factor (SDNF). J. Neurosci. 8 (3): 733-745, 1988) were used as the in vitro biological test model. Briefly, the mesencephalic tegmentum and the striatum were isolated from 14-and 15-day-old rat embryos respectively in sterile conditions. The pooled brain areas were incubated for 15 min with PBS containing 0.25% trypsin (w/v) and then for another 15 min in the presence of a soybean trypsin inhibitor (0.25%) and DNase (0.01%). The cells were separated by crushing through a syringe with a 22-gauge needle, then centrifuged, resuspended in the culture medium, passed through a 20-$\mu$m Nytex filter, counted and placed in wells (24 mm) covered in polyornithine (3 ug/ml). The neurons were cultivated in a medium containing BME and Ham's F12 (1:1 v/v) with 33 mM of glucose, 2 mM of glutamine and the following supplements, as described by Di Porzio et al. (Di Porzio U. et al.: Effect of striatal cells on in vitro maturation of mesencephalic neurons grown in serum-free conditions. Nature 288: 370-373, 1980): 25 ug/ml insulin, 100 ug/ml transferrin, 60 uM putrescine, 20 nM progesterone, 30 nM sodium selenite, 0.5 U/ml penicillin G and 0.5 ug/ml streptomycin. As a rule, 0.6 ml of culture medium containing 0.5 x 106 mesencephalic cells and 0.5 x 106 striatal cells, were added to each 24-mm well.

### High-affinity Uptake of 3H Dopamine

The uptake of 3H dopamine was effected as described by Berger et al. (Berger B. et al.: Long-term development of mesencephalic dopamin- ergic neurons of mouse embryos in dissociated primary cultures: morphological and histochemical characteristics. Neuroscience, 7: 193-205, 1982). The cells were washed twice with warm PBS to which glucose (5 mM), CaCl2 (1 mM), MgSO4 (1 mM) had been added and further incubated for 15 min in 0.8 ml of the same solution. Then 0.5 ml (50 nM final concentration, specific activity 22-33 Ci/nmol) of 3H dopamine were added and incubation was continued for 15 min at 37°C. Uptake was blocked by removing the incubation mixture followed by three rapid washes with cold PBS. The incorporated 3H DA was extracted twice from each dish (15 min each time) with 0.5 ml of 0.4 M HClO4, with absolute ethanol (3:1 v/v). Recovery was over 95%. Radioactivity was assessed after the addition of 10 ml of instagel II, using a Packard Tricarb scintillation counter (460 C model). The presence of high-affinity 3H dopamine was assessed using the specific dopaminergic uptake antagonist, that is, benztropine mesylate (1 uM).

### High-affinity 14C GABA Uptake

The mesencephalic-striatal cocultures were incubated at 37°C for 15 min with 0.1 uM 14C GABA (225 mCi-/mmole) in PBS in the presence of 2 mM of β-alanine, an inhibitor of high-affinity uptake of glial GABA. High-affinity uptake for 14C GABA, neuron-specific, was assessed using as an inhibitor, 1-2,4-diaminobutyric acid (1 mM). Washing and extraction procedures were as for 3H DA uptake.

### Indirect Immunocytochemical Colouring

Mesencephalic (1.5 x 106 cells) and striatal (1.5 x 106 cells) cells were seeded on glass cover slips coated with polyornithine, and placed in 35-mm sterile dishes. Indirect immunofluorescence in the presence of RT97 monoclonal antibodies, known to be specific for the brain neurofilament protein, was conducted as described in the literature (Anderton B. H. et al.: Monoclonal antibodies show that neurofibrillary tangles and neurofilaments share antigenic determinants. Nature 298: 84-86, 1982). Indirect immunofluorescence in the presence of mouse GFAP antiserum was conducted according to the method of Raff et al. (Raff M. C. et al.: Cell type specific markers for distinguishing and studying neurons and the major classes of glial cells in culture. Brain Res. 174: 283-308, 1979). TH immunofluorescence, specific for tyrosine hydroxylase, was performed as described by Berger et al. (Berger B. et al.: Long-term development of mesencephalic dopaminergic neurons of mouse embryos in dissociated primary cultures: morphological and histochemical characteristics. Neuroscience 7: 193-205, 1982).

### Experimental Procedure

The serum samples, heat-inactivated, (dilution range 1 to 10% v/v) were added to the cultures in vitro on the 4th day, that is, when the high-affinity dopamine uptake had reached its plateau (Leon A. et al.: Sera from parkinsonian patients contain inhibitory activity for development of fetal mesencephalic dopaminergic neurons in culture. J. Neurochem. Suppl. 44:S 137 A, 1985; Prochiantz A. et al.: In vitro maturation of mesencephalic dopaminergic neurons from mouse embryos is enhanced in presence of their striatal target cells. Proc. Natl. Acad. Sci. USA 76: 5387-5391, 1979).

24 hours later, pairs of cultures containing the same serum samples were exposed for 60 minutes to rabbit (or other) complement or complement which had previously been heat-inactivated (56°C for 30 minutes). The aforesaid uptake parameters were then assessed (specific for DA and GABA) and the results were expressed as residue fractions (RF) of each serum sample:

$$RF = \frac{CPM \text{ (specific) after exposure to complement}}{CMP \text{ (specific) after exposure to inactivated complement}}$$

Immunocytochemical staining for tyrosine hydroxylase (TH) positive cells was performed as described above, in parallel experiments following the same experimental protocol.

Lastly, the data obtained underwent statistical analysis, in which the specific uptake of DA and GABA was considered significantly reduced when the RF value decreased to less than 2.5 standard deviations (SD) of the mean RF value obtained from the neurological controls.

The groups of patients were compared by the Mann Whitney nonparametric test, Student's t-test and the Chisquare test corrected according to Yates.

Dopamine (DA) and GABA content in the mesencephalicstriatal cocultures can also be assessed by electrochemical HPLC (high pressure liquid chromatography) methods and catecholamine histofluorescence, according to Dal Toso et al. (Dal Toso R. et al.: Development and survival of neurons in dissociated fetal mesencephalic serum-free cell cultures: effect of cell density and of an adult mammalian striatal-derived neuronotrophic factor (SDNF). J. Neurosci. 8 (3): 733-745, 1988).

## RESULTS

### 1. Effect of concentrations of complement on mesencephalic-striatal cocultures

As shown in Fig. 1, incubation of the complement, at concentrations of over 1%, induces a decrease in specific uptake of both DA and GABA. In parallel, cell detachment and lysis were observed, as a morphological correlation of the phenomenon. Despite this, the addition of complement at concentrations of less than 1% (<1%) does not alter the RF values relative to either DA or GABA; likewise morphological observations (by phase-contrast microscope) reveals no signs of toxicity.

### 2. Effect of human serum on high-affinity uptake for Dopamine and GABA

Samples of inactivated serum, from both Parkinsonian patients and neurological controls, do not alter DA and GABA uptake, even at high concentrations (such as 1:10).

On the contrary (as can be seen in Fig. 2), there was a selective reduction in dopamine uptake (with no morphological evidence of cell damage), when the complement (at concentrations of 1%) was added to cultures containing serum samples (concentration 5%) from some of the parkinsonian patients. In particular, when the sera of PK patients were assigned to either the clinically determined idiopathic or vascular parkinsonism groups, 14 out of 18 patients with idiopathic parkinsonism and 3 out of 13 patients with vascular parkinsonism (Fisher test: $p < 0.01$) achieved DA-RF values less than 82% of neurologic controls and, thus (see methods), were considered seropositive (Figure 3, Table 2). The mean DA-RF was accordingly reduced by a significant extend only with sera from idiopathic patients (Table 2).

No significant differences were found among the mean values of GABA-RF obtained with sera from patients with or without vascular brain damage, or neurologic controls (Figure 3). The use of more concentrated serum (10% versus 5%) did not increase the proportion of active sera in the various groups but further decreased the DA-RF (without affecting GABA-RF) in seropositive patients (Figures 4A and 4B). Seropositive and seronegative patients did not differ in demographic and clinical features, including age, sex, age at onset, duration of illness, clinical type, severity of the disease and current medical therapy. It should be noted that the incidence of ischemic brain damage is more frequent in negative patients than in positive patients.

Indeed, evidence of basal ganglia infarctions was radio-graphically detected in 7 out of 10 seronegative patients of the vascular parkinsonian group, and in none of 3 seronegative patients of the same group. Instead, these last three seronegative patients and the remaining three seropositive patients of the vascular parkinsonian group were found to have multiple subcortical white matter lesions.

EP 0 483 014 A1

Table 2:

Statistical analysis of dopamine-RF values of neurological controls and parkinsonian patients.

|  | Neurological Controls | Parkinsonian Patients | |
|---|---|---|---|
|  |  | idiopathic | vascular |
| Number of Seropositive Patients | 0 | 14* | 3 |
| Number of Seronegative Patients | 18 | 4 | 10 |
| Mean Dopamine % Residual Fraction ± S.E.M. | 99.9 ± 17 | 64.7 ± 3.7** | 83.4 ± 6.4 |

Fisher test:  * $p < 0.01$ idiopathic vs. vascular parkinsonian patients.

Mann-Whitney test:  ** $p < 0.01$ idiopathic vs. vascular parkinsonian patients.

No correlation was observed between topography of the radiographic lesions and complement-dependent serum toxicity (i.e., serum toxicity is not dependent on lesion site).

3. Immunocytochemical staining for TH-positive cells

As shown in Fig. 5, the addition of complement (1% concentration) to cultures containing serum samples (5% concentration) from sero-positive parkinsonian patients significantly reduced the number of TH-positive neurons, relative to heat-inactivated serum or complement-treated cultures. No such effect was observed using serum samples from sero-negative parkinsonian patients or neurologic controls; thus confirming the specificity of the antigen recognized by the antibody, as indicator of a specific dopaminergic effect.

CONCLUSIONS

The data reported herein show the remarkable validity of the test for high-affinity dopamine uptake on dopaminergic neuronal cultures, as a diagnostic tool for idiopathic Parkinson's disease. It has been seen that about 77% of all idiopathic patients present complement-dependent inhibition of dopamine uptake and high cell cytotoxicity, unlike symptomatic parkinsonian patients.

This test can prove to be of considerable use at a clinical level, as it allows the specific and early

This test can prove to be of considerable use at a clinical level, as it allows the specific and early detection of a marker representing a pathological situation in the CNS. It supplies a valuable diagnostic tool able to identify a neurodegenerative disorder such as idiopathic Parkinson's disease.

It must be noted that the detection of high, complement-dependent cytotoxicity in serum by an in vitro test is possible when the pathology in question is still in its very early stages, when it cannot be diagnosed by any other means.

The present method also opens up very interesting perspectives both for the prevention of this pathology and for the identification of new, pharmacologically active molecules and relative therapies.

For purely descriptive purposes, and without limiting the invention in any way, the following are exmaples of kits for the diagnosis of idiopathic Parkinson's disease in the serum of patients. In particular, examples of kits to use on mesencephalic-striatal cells, other catecholaminergic cells or any other kind of cell which presents the marker antigen (on the basis of the results relative to the above Description are described).

Example 1:

one kit contains:

a) one 10-ul vial containing $^3$H-dopamine (di-hydroxyphenylethylamine,3,4,[7-$^3$H]) 0.74-1,48 TBq (20-40 Ci/mmol) 50 nmol/ul in 0.2N acetic acid-ethanol (9:1); to be stored under nitrogen atmosphere.

b) one 1-ml vial containing freeze-dried rabbit complement to be reconstituted with distilled water.

c) one 10-ml ampoule of saline, in sterile phosphate buffer (pH 7,4), containing glucose (5 mM), $CaCl_2$ (1 mM) and $MgSO_4$ (1 mM).

d) one 1-ml ampoule of saline, in sterile phosphate buffer (pH 7,4), containing glucose (5 mM), $CaCl_2$ (1 mM), $MgSO_4$ (1 mM) and benztropine mesylate (5 uM).

e) three dishes with multiple wells, 24 mm in diameter, coated with L-polyornithine (in sterile pack).

f) one packet containing powdered Eagle's medium and Ham's $F_{12}$ (ratio 1:1), glucose (33 mM), $NaHCO_3$ (15 mM), glutamine (2 mM), Hepes (10 mM), transferrin (100 ug/ml), sodium selenite (30 nM), penicillin (0.5 U/ml), streptomycin (0.5 ug/ml), in a sufficient quantity for 50 ml of liquid culture medium.

g) one 10-ml vial containing insulin (1.25 mg) to be dissolved in 100 ul of 0.1N HCl and diluted to 10 ml with culture medium and then to be added to the remaining 40 ml of medium.

h) one 1-ml vial containing progesterone (1 nmole) to be diluted in 0.1 ml of absolute ethanol and added to the 50 ml of medium.

Example 2:

a kit containing:

a) one 10-ul vial containing $^3$H-dopamine (di-hydroxyphenylethylamine,3,4,[7-$^3$H]) 0,74- -1,48 TBq (20-40 Ci/mmole) 50 nmole/ul in 0,2N acetic acid-ethanol (9:1); to be stored under nitrogen.

b) one 1-ml vial containing freeze-dried rabbit complement to be dissolved in distilled water. c) one 10-ml ampoule of saline, in sterile phosphate buffer (pH 7,4), containing glucose (5 mM), $CaCl_2$ (1 mM) and $MgSO_4$ (1 mM).

d) one 1-ml ampoule of saline, in sterile phosphate buffer (pH 7,4), containing glucose (5 mM), $CaCl_2$ (1 mM), $MgSO_4$ (1 mM) and benztropine mesylate (5 uM).

e) 3 multi-well dishes, each well measuring 24 mm in diameter, coated in L-polyornithine (in sterile pack).

f) one packet containing powdered Eagle's culture medium and Ham's $F_{12}$ (ratio 1:1), glucose (33 mM), $NaHCO_3$ (15 mM), glutamine (2 mM), Hepes (10 mM), transferrin (100 ug/ml), sodium selenite (30 nM), penicillin (0.5 U/ml), streptomycin (0.5 ug/ml), in a sufficient quantity for 50 ml of liquid culture medium.

g) one 10-ml vial containing insulin (1.25 mg) to be dissolved in 100 ul of 0.1N HCl and diluted to 10 ml with culture medium and then added to the remaining 40 ml of medium.

h) one 1-ml vial containing progesterone (1 nmole) to be diluted in 0.1 ml of absolute ethanol and added to the 50 ml of medium.

i) one 10-ml vial containing gamma-aminobutyric acid [1-$^{14}$C] 226 mCi/mmole, 100 nmole/ul in absolute ethanol.

l) one 1-ml ampoule containing saline in sterile phosphate buffer (pH 7.4), containing glucose (5 mM), $CaCl_2$ (1 mM), $MgSO_4$ (1 mM) and L-2,4-diaminobutyric acid (1 mM).

Example 3:

a kit for the immunochemical evaluation of cytotoxicity in sera from patients with Parkinson's disease, containing:

a) one 1-ml ampoule containing mouse monoclonal antibody anti-tyrosine hydroxylase, freeze-dried and stabilized, to be reconstituted with distilled water.

b) one 1-ml vial containing goat polyclonal antibody, anti-mouse IgG, affinity-purified and conjugated with isothyocyanate fluorescein, freeze-dried and stabilized, to be made up with distilled water.

c) ten plastic Petri dishes, 35 mm in diameter, with 10 glass cover slips coated with polyornithine (in sterile packs).

d) one packet containing powdered Eagle's culture medium and Ham's $F_{12}$ (ratio 1:1), glucose (33 mM), $NaHCO_3$ (15 mM), glutamine (2 mM), hepes (10 mM), transferrin (100 ug/ml), sodium selenite (30 nM), penicillin (0.5 U/ml), streptomycin (0,5 ug/ml), in a sufficient quantity for 50 ml of liquid culture medium.

e) one 1-ml ampoule of freeze-dried rabbit complement to be reconstituted with distilled water.

The aforesaid kits are to be used as reported hereinabove wherein materials and methods are described.

## Claims

1. An in vitro method for diagnosing Parkinson's disease in a human patient, which comprises the steps of:
   (a) culturing cells in the presence of dopamine and serum of a patient to be tested for Parkinson's disease; and
   (b) measuring high-affinity dopamine uptake by said cells.

2. The method according to claim 1, comprising the steps of:
   culturing said cells in the presense of dopamine, serum and complement; and
   measuring high-affinity dopamine uptake by said cells.

3. The method according to claim 1 or 2, wherein said dopamine is labelled and the presence of said labelled dopamine is measured (detected) in cells.

4. The method according to claim 3, wherein said dopamine is radioactively labelled.

5. The method according to claim 4, wherein said radioactively labelled dopamine is $^3$H dopamine.

6. The method according to any one of claims 1 to 5, in which said cells are mesencephalic dopaminergic neurons expressing neurotransmitter-related properties.

7. The method according to claim 6, in which said cells are a coculture of mesencephalic and striatal neurons.

8. The method according to any one of claims 1 to 7, in which said patient has idiopathic Parkinson's disease.

9. The method according to claim 8, wherein said cells are cultured in the presence of GABA, radioactively

labelled with $^{14}$C, and wherein high-affinity specific $^{14}$C GABA uptake is measured in said cells.

10. An in vitro method for diagnosing Parkinson's disease in a human patient, which comprises the steps of:
culturing cells in the presence of complement and serum of a patient to be tested for Parkinson's disease; and
immunocytochemically assessing the properties of said cells.

11. The method according to claim 10, in which said cells are mesencephalic dopaminergic neurons expressing neurotransmitter-related properties.

12. The method according to claim 11, in which said cells are a coculture of mesencephalic and striatal neurons.

13. The method according to any one of claims 10 to 12, in which the immunocytochemical properties of the dopamiminergic cells are assessed by an antibody for tyrosine hydroxylase.

14. The method according to any one of claims 10 to 13, in which the immunocytochemical properties of said cells are used to reveal the presence of antibodies in said serum specific for surface antigens of said cells.

15. The method according to any one of claims 1 to 14, to be used for the identification of new pharmacologically active molecules.

16. The kit of the diagnosis of Parkinson's disease containing :
a first receptacle containing labelled dopamine; and
a second receptacle containing complement.

17. The kit according to claim 16, wherein said labelled dopamine is $^{3}$H dopamine.

18. The kit according to claim 16 or 17, which further comprises single or multiple dishes or plates, substrate and culture medium for cultivating neuronal cells.

19. The kit according to claim 18, wherein said neuronal cells are mesencephalic dopamine neurons which express neurotransmitter-related properties.

20. The kit according to claims 18 or 19, wherein said neuronal cells to be cultivated are a coculture of mesencephalic-striatal neurons.

Serum-free Mesencephalic
Striatal Coculture

Mean±S.E.M. of 3 experiments: S.E.M.<10%

FIGURE 1

FIGURE 2

FIGURE 3

## NEUROLOGIC CONTROL

Figure 4B

## PARKINSONIAN PATIENT

FIGURE 4A

FIGURE 5

**European Patent Office**

**EUROPEAN SEARCH REPORT**

Application Number

EP 91 40 2859

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y,D | JOURNAL OF NEUROCHEMISTRY vol. 44, no. SUPP, 1985, NEW YORK NY USA page 137A; A.LEON ET AL.: 'Sera from parkinsonian patients contain inhibitory activity for development of fetal mesencephalic dopaminergic neurons in culture.' See whole abstract. --- | 1-20 | G01N33/50 G01N33/573 G01N33/94 |
| Y | EP-A-0 324 037 (C. AROONSAKUL.) * page 2, column 2, line 13 - page 3, column 4, line 54 * ----- | 1-20 | |
| | | | **TECHNICAL FIELDS SEARCHED (Int. Cl.5 )** |
| | | | G01N |

The present search report has been drawn up for all claims

| Place of search | Date of completion of the search | Examiner |
|---|---|---|
| THE HAGUE | 03 FEBRUARY 1992 | VAN BOHEMEN C.G. |

EPO FORM 1503 03.82 (P0401)